Europäisches Patentamt

(19) European Patent Office

Office européen des brevets

(11) Publication number: **0 202 159 B1**

## (12) EUROPEAN PATENT SPECIFICATION

(45) Date of publication of patent specification :
03.07.91 Bulletin 91/27

(51) Int. Cl.⁵: **A61M 31/00, A61K 9/22**

(21) Application number : **86400977.4**

(22) Date of filing : **06.05.86**

---

(54) **Drug delivery device which can be retained in the stomach for a controlled period of time.**

---

(30) Priority : 10.05.85 US 732333
29.05.85 US 738633
29.05.85 US 738634

(43) Date of publication of application :
20.11.86 Bulletin 86/47

(45) Publication of the grant of the patent :
03.07.91 Bulletin 91/27

(84) Designated Contracting States :
CH DE FR GB IT LI NL

(56) References cited :
EP-A- 0 010 987
WO-A-86/00538
FR-A- 2 526 306
US-A- 3 844 285
US-A- 3 944 064

(73) Proprietor : **MERCK & CO. INC.**
**126, East Lincoln Avenue P.O. Box 2000**
**Rahway New Jersey 07065-0900 (US)**

(72) Inventor : **Gardner, Colin R.**
**525 Lawrence Avenue**
**Lawrence Kansas 66044 (US)**
Inventor : **Caldwell, Larry J.**
**2709 Princeton Boulevard**
**Lawrence Kansas 66044 (US)**
Inventor : **Cargill, Robyn C.**
**937 Alabama**
**Lawrence Kansas 66044 (US)**
Inventor : **Higuchi, Takeru**
**2811 Schwarz Road**
**Lawrence Kansas 66044 (US)**

(74) Representative : **Ahner, Francis et al**
**CABINET REGIMBEAU, 26, avenue Kléber**
**F-75116 Paris (FR)**

EP 0 202 159 B1

## Description

DESCRIPTION OF THE PRIOR ART

Numerous patents and publications have described devices which can be retained in the stomach for extended periods of time. The prior art can be categorically described according to the technologies listed below :

1.) Floating devices
2.) Swelling devices
3.) Inflating balloons and
4.) For ruminant animals--various shapes.

There follows a discussion of each of the above technologies which demonstrates the technological advancement of the invention over the prior art :

(1) Michaels et al (Alza Corporation), U.S. Patent No. 3,901,232, describe a device which contains a drug delivery compartment attached to an envelope which is contained within an erodible capsule. Upon ingestion, the capsule dissolves and a liquid contained in the envelope vaporizes and swells the envelope to provide a means of retention by flotation within the desired body cavity e.g. the stomach.

S. Watanabe et al, U.S. Patent No. 3,976,764 teach the use of a hollow or low density core surrounded by polymeric materials containing drug(s). The device floats in the stomach and releases said drug over an extended period of time.

Sheth and Tossounian, U.S. Patent Nos. 4,140,755 and 4,167,558 describe the use of a tablet which is hydrodynamically balanced to be buoyant under gastric conditions thereby remaining in the stomach for an extended period of time.

All of the references in this category function on the basis of floatation or buoyancy and do not teach the use of size to retain them in the stomach.

(2) Johnson et al, U.S. Patent No. 3,574,820 teach the use of tablets or capsules containing a reaction product of gelatin and N-acetyl-homocysteine thiolactone as a component of an oral dosage form small enough to be swallowed but which swells in the stomach to become too large to pass through the pylorus.

Banker, U.S. Patent No. 261,242 describes the use of a swellable polymer (e.g. Gantrez) coated on the outside of tablets or capsules such that the coating swells under gastric conditions.

Mamajek and Moyer, U.S. Patent No. 4,207,890 teach the use of an expandable envelope containing a drug and an agent which expands when gastric fluid permeates through the envelope. The device enlarges and is retained in the stomach for an extended period. There is no teaching as to how the device disintegrates or what controls its exit from the stomach.

Theeuwes and Urquhart, U.S. Patent No. 4,434,153 describe the use of a device (containing a hydrogel) which can enter into the gastrointestinal environment where it imbibes fluid and swells 2-50 fold so that it is retained in the stomach over an extended period. Small pills containing drug are released from this device and subsequently delivered for gastric or intestinal absorption.

The patents of this category teach the use of size to retain the device in the stomach and function by absorption of gastric fluid to cause swelling of an expandable polymeric material. No indication of the obtainable duration is given or of control of the process by which the device disintegrates or otherwise is expelled from the stomach.

(3) Alza Corporation, U.S. Patent Nos. 3,797,492 and 3,901,232 and U.S. Patent No 3 944 064 disclose the use of inflatable bags containing a gas or vapor-generating system to cause inflation following release of the device from a bioerodible capsule in the stomach (or other desired body cavity). The inflated envelope causes the device to be retained in the desired site (e.g. stomach) and drug is released from an attached delivery system. When all the drug has been expelled the envelope deflates and the device passes from the stomach.

These patents imply the use of size to retain the device in the stomach and achieve enlarged size in the stomach via conversion of liquid (or solid) components to a gaseous form. They do not teach the use of mechanical movement to create large forms of the device from a smaller configuration.

(4) R. Laby, U.S. Patent No. 3,844,285 describes the use of a device having one initial configuration or adapted to be arranged in the configuration so as to pass into the rumen of an animal, whereupon, it changes to a second configuration which will prevent or hinder regurgitation of the device. This patent is applicable to devices which are delivered to the rumen and required to remain in the rumen by preventing regurgitation. The reference does not teach devices designed to enter and be retained in the stomach of humans or other non-ruminant animals. Neither does it teach any procedure for preventing passage of such devices through the pylorus of humans or non-ruminant animals since only regurgitation is hindered or prevented by the device of the reference. The anatomy of the rumen is quite different from that of the stomach of non-ruminant species.

## BACKGROUND OF THE INVENTION

The invention provides a drug delivery device comprising at least one drug and a continuous solid-stick figure, a planar figure or ring figure made from polymer(s) that is retained in the stomach for predictable and extended periods of time for releasing therapeutic or other beneficial agents.

Many orally administered drugs fail to achieve their full potential because of a number of problems including :

a. Slow and incomplete intestinal absorption.

b. Existence of preferential absorption sites in the gastrointestinal tract (absorption windows).

c. Short biological half-life (in particular if the therapeutic index is low).

Solutions to the delivery problems for these agents cannot be guaranteed using conventional controlled release technology since the site of drug release may be beyond the site of optimal absorption or the transit time through the asbsorbing portion of the gastrointestinal tract may be too short to effect an increase in the duration of action of the drug. In order to optimize the delivery of these agents to achieve maximum effectiveness (and reduce concentration-related side effects) it is desirable to obtain a drug delivery device which would be retained in the stomach for a prolonged, predictable period of time during which it would release the agent in a predetermined pattern. At the end of its period of usefulness, the device would disintegrate or otherwise alter its properties such that it would exit from the stomach and pass down the intestine.

Accordingly, it is an object of the invention to provide a means of retaining the dosage form in the stomach for an extended, predictable period of time. Thereby the duration of absorption and hence the duration of action of drugs with short biological half-lives could be extended. Likewise the bioavailability of the agents could be improved over that achieved from a conventional dosage form or conventional sustained release preparation.

Another object of the invention is to provide a mechanism whereby after a predetermined time the device will erode, disintegrate or otherwise alter its properties and pass out of the stomach and into the intestine. In addition the device should not lodge in the intestine thereby causing an obstruction.

A further object is to devise a device which will not obstruct the passage of food while the device is in the stomach or after it has passed into the intestine.

Other objects, features and advantages of the invention will be apparent to one skilled in the art from the detailed description of the invention which follows.

Immediately after consumption of food, the stomach acts as a holding tank for the solids while they are digested and broken down into small particles (1-2 mm in diameter) by the action of acid, enzymes and the physical grinding of the food particles by muscular contractions of the stomach wall. Due to the sieving action of the pyloric valve only particles of the order of 1-2 mm leave the stomach and pass into the intestine during the digestive period. Approximately two hours after digestion of a meal, the stomach passes into its interdigestive phase during which there are regular and frequent (every 1-1/2-2 hours) periods of intense contraction known as the interdigestive migratory myoelectric complex (IMMC). These contractions which can apply forces up to 200 cm of water (approximately 0.2 atmospheres pressure) are designed to remove any remaining ingested food from the stomach through a fully open pylorus. Clearly, then, any dosage form which is designed to remain in the stomach for an extended period (i.e., greater than a few hours) must be capable of withstanding these contractive forces to prevent it from passing from the stomach into the intestine.

## A BRIEF DESCRIPTION OF THE DRAWINGS

Figure 1 is a preferred configuration of a continuous solid-stick figure device in a tetrahedral form with fastened ends.

Figure 1a is a preferred configuration of the device in a tetrahedral form.

Figure 1b is formed when corner 1 and 3 and corners 2 and 4 of Figure 1a are compressed together.

Figure 1c refers to encapsulation of Figure 1(b).

Figure 2 is a preferred configuration of a planar figure device in a 4-lobed planar form.

Figures 2a and 2b show the device of figure 2 folded and inserted into a capsule for oral administration.

Figure 3 is a preferred configuration of the device in a planar, disc-shaped form.

Figures 4a and 4b show the device of figure 3 folded and inserted into a capsule for oral administration.

Figure 5 is a preferred configuration of the device in a planar, 4-limbed cross form. Each limb of the device contains a rigid, soluble polymer which slowly erodes resulting in loss of integrity of the device.

Figures 6a and 6b show the device of figure 5 folded and inserted into a capsule for oral administration.

Figure 7 is a preferred configuration of a ring figure device in a ring form.

Figure 8a shows the device in a folded configuration.

Figure 8b refers to encapsulation of Figure 8a.

## DESCRIPTION OF THE INVENTION

The invention is directed to a gastric retention device comprising a continuous stick figure prepared from at least one erodible polymer, said device having the following properties :

a) compressible to a size, allowing its insertion into a retaining capsule or like container designed to dissolve within the stomach after oral ingestion, and suitable for swallowing ;

b) expandable, after dissolution and/or desintegration of said retaining capsule, in its original shape and to size which will prevent passage through a pylorus for a predetermined time ;

c) sufficiently resistant to contractive forces exerced by the stomach, in particular simultaneous forces in two directions acting to reduce the device to a size less large than the pyloric valve, in order to prevent passage through a pylorus for a predetermined time ; and

d) erodible in the presence of gastric juices so that said device after a predetermined time is no longer able to retain or attain the expanded configuration defined in (b) above and/or resist a simultaneous force in two directions as defined in (c) above.

The invention can also be used in a method for the controlled release over a period of time of a drug in the stomach, comprising a gastric retention device in the form of a continuous stick figure prepared from at least one erodible polymer, said device having the following properties :

a) compressible to a size, allowing its insertion into a retaining capsule or like container designed to dissolve within the stomach after oral ingestion, and suitable for swallowing ;

b) expandable, after dissolution and/or desintegration of said retaining capsule, in its original shape and to size which will prevent passage through a pylorus for a predetermined time ;

c) sufficiently resistant to contractive forces exerced by the stomach, in particular simultaneous forces in two directions acting to reduce the device to a size less large than the pyloric valve, in order to prevent passage through a pylorus for a predetermined time ; and

d) erodible in the presence of gastric juices so that said device after a predetermined time is no longer able to retain or attain the expanded configuration defined in (b) above and/or resist a simultaneous force in two directions as defined in (c) above.

## Definitions :

Gastric retention device is a device which resides in the confines of the stomach for the purpose of providing a platform for controlled release of biologically active agents.

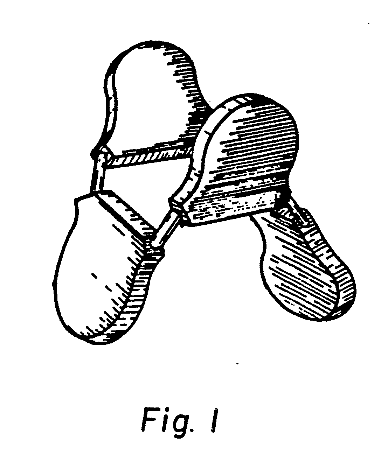

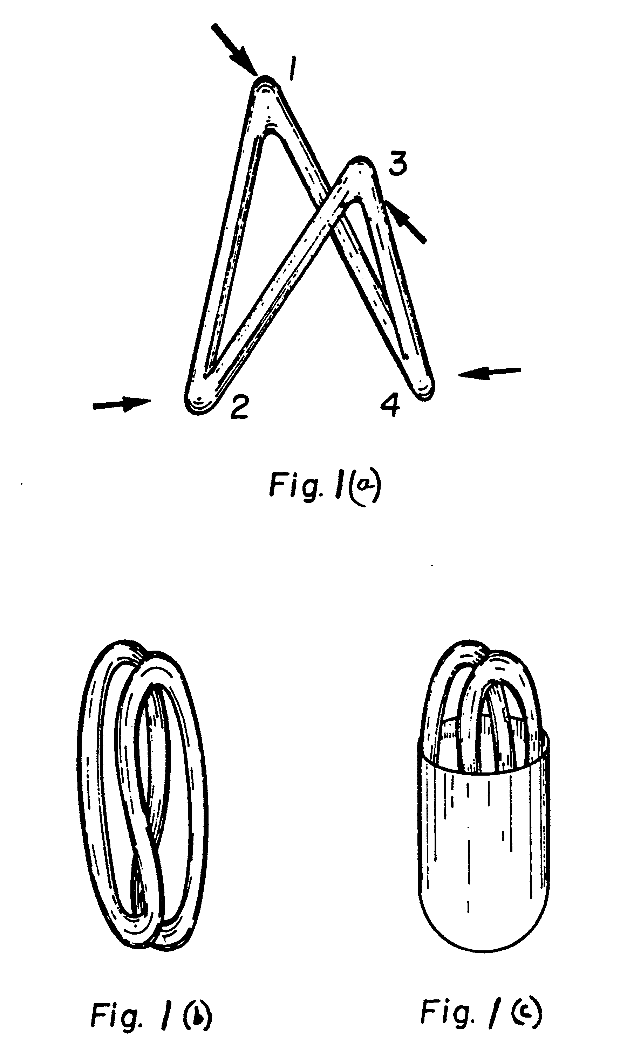

Continuous solid-stick figure refers to a framework composed of more or less rigid rods or sticks (preferably a Tensile Modulus of at least $1 \times 10^3$ to $50 \times 10^6$ psi, more preferably in the range of $1.5 \times 10^4$ to $20 \times 10^6$ psi) which are bent and/or fastened together in a manner such that the framework will not pass out of the stomach, but will allow passage of food. Preferred configurations are illustrated in Figures 1, 1a, 1(b) and 1(c).

The expanded tetrahedral device shown in Figures 1 is compressed by squeezing together corners 1 and 3 and corners 2 and 4 of Figure 1(a) by applying forces at right angles to subsequently obtain the form of Figure 1(b) and ultimately inserted into a hard gelatin capsule as illustrated in Figure 1(c).

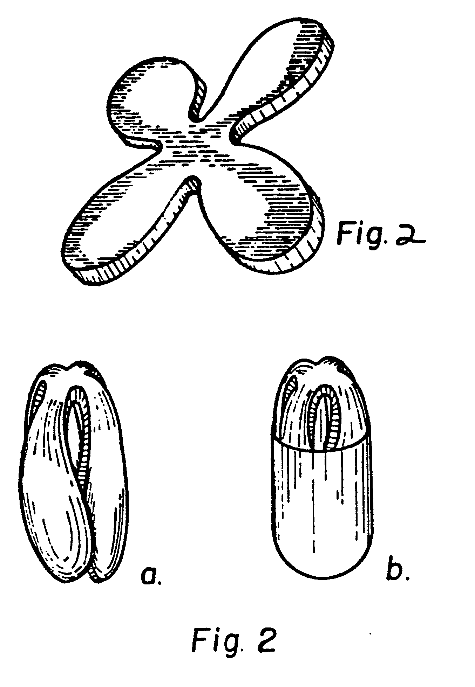

A planar disc-shaped or multi-lobed flat or planar device may be used which is large enough and rigid enough such that said device will not pass out of the stomach but will allow passage of food around said device. Preferred configurations are illustrated in Figures 2-6.

The longest diamerter (the device need not be symmetrical) of the device may vary between 1.6 and 5 cm; more preferably in the range 2.0 to 4 cm ; and most preferably in the range 3.2 to 3.6 cm.

The expanded devices shown in Figures 2, 3 and 5 are compressed by folding or rolling to subsequently obtain the form of Figures 2a, 4a, and 6a respectively and ultimately inserted into capsules as illustrated in Figures 2b, 4b and 6b.

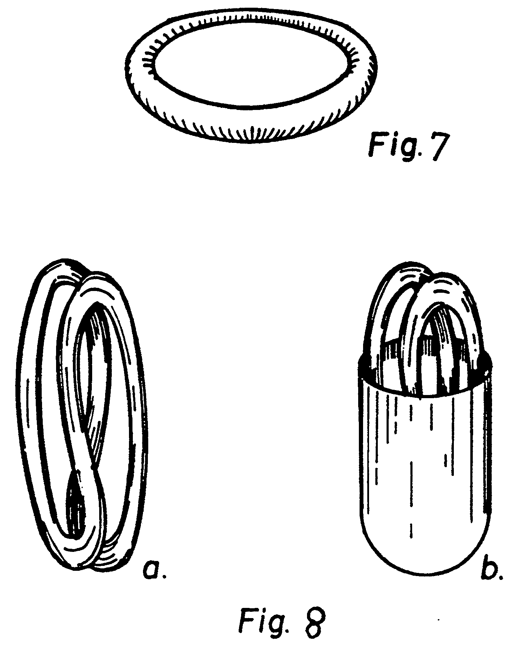

A ring refers to an annulus as shown in Figure 7. The ring is preferably made of a non-erodible extruded rod-shaped material and then merely joining the ends with an erodible material. However, the whole ring may be made of erodible material.

The diamerter of the ring may vary between 1.6 and 5 cm ; more preferably in the range 2.5 to 4 cm ; and most preferably in the range 3.2 to 3.6 cm.

The cross-section of the band making up the circumference of the annulus may be one of many shapes (e.g.., circular, rectangular, square, triangular and the like). The dimensions of the cross-section should be in the range 0.5 to 5 mm, more preferably in the range 1 to 3 mm. The cross-section may be solid or hollow ; i.e., the ring may be constructed from a tube which may be empty or may contain liquid, solid or gaseous material.

The ring shown in Figure 7 may be folded as shown in Figure 8a and inserted into a capsule (Figure 8b) for oral administration.

Criterion A means that the desired expanded configuration of said device is too large to be swallowed and thus must be compressed and contained in a conventional capsule or like container for swallowing. The capsule is designed to dissolve after oral ingestion within the confines of the stomach.

Criterion B means that after the compressed device within the capsule container reaches the stomach, upon dissolution and/or disintegration of the retaining capsule or like container, said device will expand or unfold in such a manner that results in its original shape which is too large to pass from the stomach into the intestine unless enough force is applied to recompress the device. This "expanded" form of the device will remain in the stomach for a pre-determined period of time depending on the desired time profile of release of the biologically active agent, which is a part of the device or contained within a controlled release module that is attached to the retention device. The "pre-determined time" is preferably within the range of 1 hour to 1 year. For example, a biologically active agent which prevents heart-worm infestation in dogs might require gastric retention and controlled delivery of said agent over the course of 6-12 months. This period depends on the seasonal changes of the mosquito population which is the main vector of heart worm infection in dogs. A second example is the use of such a gastric retention device to maintain controlled delivery of a contraceptive agent. The desired retention time of the device might be 3-4 weeks to coincide with the normal frequency of menses. A third example is the use of such a device to achieve once daily dosing, if such device is coupled to a controlled release device for delivery of an agent with a very short half-life of biological activity, such as a dopamine agonist for treatment of Parkinsonism.

Criterion C means that the device in question must sustain some compressive force, in excess of that amount the stomach is able to apply, in order for said device to not prematurely pass on into the intestine. For humans and animals with similar gastric anatomy such as dogs, the gastric pouch is capable of applying forces of 50-150 grams.

Criterion D means that after some predetermined time, during which said device has been utilized, some part of the device will suddenly or gradually fail to meet Criteria B and/or C. That is, the device will either be unable to withstand a lesser compressive force, or will lose its integrity as a single unit, and thereby be subject to the normal propulsive forces and pass out of the stomach. By doing so, the danger of multiple devices causing obstructive problems, is obviated.

These devices can be manufactured by a number of processes including injection molding, extrusion, film-forming, laminating and the like as will be clear to one skilled in the art. For example, a mold can be constructed in the desired shape of the device and filled with appropriate material(s) in the liquid state and then allowed to cure by chemical processes or cooling if thermosetting materials(s) is used.

A critical property of the devices is that, after a certain, pre-determined period of time in the stomach they will alter their properties in a manner which will permit their elimination from the stomach into the intestine. For this purpose the devices must contain components(s), sections(s) or points(s) which will erode or dissolve in the aqueous environment of the stomach.

For example, the four lobes of the device of figure 2 may be fastened at a central point by glue (or other material) which will dissolve or erode during the desired time period, thereby liberating the individual lobes which will then easily pass out of the stomach.

The disc can be manufactured from composite mateirals such that one or more components(s) will erode or dissolve leaving a disc whose mechanical properties are such that it can be readily removed from the stomach by the compression forces associated with the recurrent interdigestive migratory myoelectric complex.

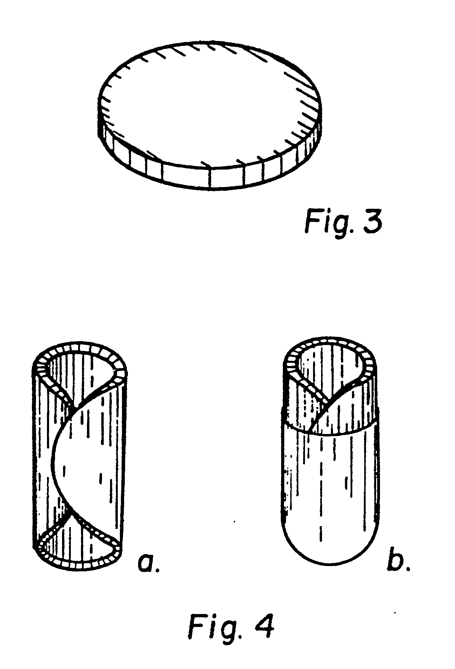

A further modification of the four-lobed form is to have contained within the lobes an erodible or dissolving structure which will maintain the lobes in a rigid configuration until the internal, structurally supporting material has disintergrated. At that point the device would be incapable of maintaining its rigid, planar conformation and would be eliminated from the stomach. (see figure 3 and Example 11).

Although the polymers may be blends, combinations, composites or copolymers (erodible and non-erodible), said polymers must be erodible at the point(s) of desired dissolution and/or disintegration. Representative erodible polymers which can be employed in the practice of the invention are cellulosics such as Klucel (hydroxypropylcellulose), cellulose acetate phthalate, methyl cellulose, hydroxypropylmethyl-cellulose phthalate and the like ; ethylene/vinyl alcohol copolymer ; ethylenemaleic anhydride copolymer ; polyacrylates such as Eudragit E (cationic copolymer based on dimethylaminoethyl methylacrylate and neutral methylacrylic acid esters(, poly(acrylic acid), poly(methacrylic acid) and the like ; polylactones such as poly(caprolactone) and the like ; polyanhydrides such as poly[bis-(p-carboxyphenoxy)-propane anhydride], poly(terephthalic acid anhydride) and the like ; polyvinyl pyrrolidone ; polyamides and polypeptides such as polylysine, polyglutamic acid and the like ; gelatin and derivatives such as those produced on reaction with N-acetyl-homo-cysteine thiolactone and the like ; polyesters such as polylactides, polyglycolides, poly(betahydroxybutyric acid) and the like; poly(ortho esters) such as copolymers of DETOSU with diols such as hexane diol, decane diol, cyclohexane dimethanol, ethylene glycol, polyethylene glycol and incorporated herein by reference those poly(ortho) esters

5

described and disclosed in U.S. Patent No. 4,304,767 and the like ; polyurethanes such as those prepared with poly(tetramethylene oxide), prepolymer terminated with hexamethylene diisocyanate and a poly functional diester of oxalic acid and glycerol, di-(2,3-dihydroxypropyl)oxalate or its mixture with 3,6-dioxaoctane-1,8-diol and the like ; polyacrylonitriles such as poly(alkyl-α-cyanoacrylates) wherein the alkyl is methyl, ethyl, propyl, butyl, amyl and the like ; and types of inorganic glass based on polyphosphates and fused salts.

Representative non-erodible polymers that may be employed in the practice of the invention are polyolefins such as polyethylene, polypropylene, ethylene vinyl acetate copolymers, poly(tetrafluoro-ethylene) and the like; rubbers such as silicon based rubber, styrene-butadiene copolymers and the like polyamides such as nylon 6,6, nylon 6, and the like ; polyesters such as poly(ethylene terephthalate) and the like ; polyurethanes formed from diisocyanates such as 1,6-hexane diisocyanate or biphenylene diisocyanate etc. and diols such as ethylene glycol, 1,4-butane diol and the like ; and cellulosics such as ethyl cellulose, cellulose diacetate, cellulose triacetate and the like.

A drug or medicament may be associated with the gastric retention device in different ways, depending on the physical and chemical properties of the drug or medicament. For example, the drug may be dispersed as a solution or suspension within an erodible polymer matrix such that as the matrix erodes within the confines of the gastric pouch, the drug is released at a predetermined rate. Similarly, the drug may be dissolved or dispersed within a non-erodible matrix material comprising part of the retention device. As this matrix comes in contact with gastric fluid, the drug diffuses out of the non-erodible matrix at a predetermined rate. An alternative to incorporating the drug as an integral part of the gastric retention device is to simply fasten a controlled release drug module to the retention device. Such a module might be a miniature constant-flow pump, either mechanically or osmotically driven, and may be fastened to the retention device by gluing or tethering. Likewise it may consist of a matrix system, erodible or non-erodible, fastened to the retention device.

In man and similarly-sized non-ruminate mammals, the size of the pyloric valve between the stomach and small intestine is generally in the range of 3-5 cm maximum inner circumference. Objects with a mimimum circumference of more than 5 cm will generally not pass from the stomach through the pylorus. The stick-figure, planar figure and ring figure devices described herein are designed to present a minimum circumference of more than 5 cm after deployment in the stomach. Before deployme in the stomach the minimum circumference is less than 2 cm.

To further set forth the concept of the invention several gastric retention studies are shown below.

Studies were performed with Beagle dogs in order to ascertain gastric retention time of the drug delivery device described herein. The parameters investigated were size, erodibility and polymer flexibility. These devices were administered in gelatin capsules and their position in the gastro-intestinal tract determined using x-ray techniques. Each device was tested in three to four different dogs in three fed states : fed (fed 10 minutes before dosing and food ad lib thereafter) fed/fast (fed 10 minutes before dosing, and fasted thereafter for 36 hours), and fast (fasted 18 hours before dosing and thereafter for 36 hours).

The results for a tetrahedral device of different dimensions are shown below.

| Shape | Dimensions | Average % Retained 24 hours | N* |
|---|---|---|---|
| Tetrahedron | 2.0 cm (wide) x 2.0 cm (long) x 2.0 cm (high) | 92 | 12 |
| | 1.5 cm x 1.5 cm x 1.5 cm | 85 | 12 |

*Number of trials.

Polymer flexibility studies were also carried out in Beagle dogs. One point five millimeter (1.5 mm) diameter rods having varying flexibilities were extruded. These rods were constructed of polyethylene alone, polyethylene blended in various proportions with a copolymer of ethylene (86%) and vinyl acetate (16%) or the copolymer of ethylene and vinyl acetate alone. All rods also contained 15% by weight of barium sulfate to render them visible by x-ray in the g.i. tract of the dogs.

| Shape | Material | Tensile Modulus (psi) | % Retained at 24 hours | N |
|-------|----------|-----------------------|------------------------|---|
| | 100% PE | 11,556 | 92 | 12 |
| Tetrahedron | PE 94%/EVA 6% | 11,383 | 57 | 7 |
| (2 cm) | PE 30%/EVA 70% | 9,512 | 50 | 4 |
| | 100% EVA | 3,828 | 50 | 4 |

EVA composition 86% ethylene, 14% vinylacetate PE is polyethylene.

Studies were conducted on the degree of erodibility of the polymeric materials described within the invention. A series of poly(ortho ester)/polyethylene (POE/PE) blends have been tested in Beagle dogs using a modified tetrahedron device. The tetrahedron shape was formed from four silastic, 15% barium-load corners with openings for insertion of the poly(ortho ester)s/polyethylene rods. The corner pieces were 3 mm thick and formed an equilateral triangle. The completed tetrahedron measured 2 × 2 × 2 cm (see figure 1). All dogs were tested in the fasted state (fasted 18 hours before dosing and thereafter for 36 hours) and the following data obtained.

| **POE/PE | % Retained at 24 hours | N |
|----------|------------------------|---|
| 50/50 | 80 | 5 |
| 65/35 | 80 | 5 |
| 75/25 | 100 | 5 |
| 80/20 | 0 | 4 |
| 85/15 | 25 | 4 |
| 90/10 | 0 | 5 |

**POE used was prepared from cis-trans cyclohexanedimethanol and 3,9-Bis(ethylidenyl)-2,4,8,10-tetraoxaspiro [5,5]undecane [Detosu].

In vitro dissolution studies were carried out with the poly(ortho ester)/polyethylene blends. One inch pieces of the 1.5 mm diameter rod blends were tested in pH 1.5 buffer, in a 37°C water/shaker bath. At selected time intervals a piece was removed from the water/shaker bath and measured gravimetrically. Percent erosion was calculated from weight loss as shown below.

### In Vitro Dissolution - % Poly(ortho ester) Eroded

|  | Poly(ortho ester)/Polyethylene | | | | |
|---|---|---|---|---|---|
| Time (hours) | 50/50 | 65/35 | 75/25 | 80/20 | 85/15 |
| 8 | 35.4 | 40.9 | 53.2 | 95.1 | 99.3 |
| 16 | 55.2 | 77.2 | 86.9 | 97.9 | 97.3 |
| 24 | 72.0 | 84.0 | 84.4 | 98.8 | 96.6 |
| 28 | 74.6 | 90.5 | 86.4 | 98.1 | 96.8 |
| 32 | 87.4 | 88.2 | 87.5 | 98.3 | 98.4 |

The experimental error is about ±10%,

Studies were performed with Beagle dogs in order to ascertain gastric retention time of the drug delivery device described herein. The parameters investigated were size, erodibility and polymer flexibility. These devices were administered in gelatin capsules and their position in the gastrointestinal tract determined using x-ray techniques. Devices were tested in three to four differing dogs which were fasted 18 hours before dosing and thereafter for 36 hours. The effect of size (ring diameter) on gastric retention time is shown in Table I below.

### TABLE I

| Shape | Dimensions | Average % Retention at 24 Hours | N* |
|---|---|---|---|
| Ring | 3.6 cm diameter | 100 | 6 |
| Ring | 2.5 cm diameter | 70 | 10 |

*N=Number of trials

Polymer flexibility studies were also carried out in Beagle dogs. One point five millimeter (1.5 mm) diameter rods having varying flexibilities were extruded. These rods were constructed of polyethylene alone, polyethylene blended in various proportions with a copolymer of ethylene (86%) and vinyl acetate (16%) or the copolymer of ethylene and vinyl acetate alone. These rods were formed into rings and inserted into capsules for in vivo testing. Rings were also constructed from Silastic™ (Dow Company) medical grade silicone rubber. All rings also contained 15% by weight of barium sulfate to render them visible by x-ray in the g.i. tract of the dogs. See Table II below.

## TABLE II

| Shape | Material | Tensile Modulus (psi) | % Retained at 24 hours | N |
|---|---|---|---|---|
| | 100% PE | 11,556 | 100 | 6 |
| Rigid Ring | PE 94%/EVA 6% | 11,383 | 100 | 4 |
| (3.6 cm) | PE 30%/EVA 70% | 9,512 | 75 | 4 |
| | 100% EVA | 3,828 | 0 | 4 |
| Soft Ring (3.0 cm) | silicone rubber | ND* | 67 | 9 |

EVA composition 86% ethylene, 14% vinylacetate PE is polyethylene
*N,D. = not determined. This ring was very flexible.

To demonstrate that a ring could rapidly exit from the stomach when its integrity was lost, unsealed rings (i.e. strings) were administered to dogs in gelatin capsules and then gastric retention measured by X-ray techniques. The data are shown in Table III below :

## III

| Shape | Material | Size | Gastric Retention Time (hours) | N |
|---|---|---|---|---|
| String | Silastic | 6 cm long 1.5 mm diameter | 40-70 | 2 |

These results demonstrate that if a ring contains an erodible section which can be ruptured at a predetermined time, the resultant string will be eliminated from the stomach.

The erodible section(s) can be constructed from many different types of material. Examples of erosion profiles of some such materials (blends of polyethylene and a poly(orthoester)) are shown in the Table IV below.

## In Vitro Dissolution - % Poly(ortho ester) Eroded

| Time (hours) | Poly(ortho ester*)/Polyethylene | | | | |
|---|---|---|---|---|---|
| | 50/50 | 65/35 | 75/25 | 80/20 | 85/15 (% by weight) |
| 8 | 35.4 | 40.9 | 53.2 | 95.1 | 99.3 |
| 16 | 55.2 | 77.2 | 86.9 | 97.9 | 97.3 |
| 24 | 72.0 | 84.0 | 84.4 | 98.8 | 96.6 |
| 28 | 74.6 | 90.5 | 86.4 | 98.1 | 96.8 |
| 32 | 87.4 | 88.2 | 87.5 | 98.3 | 98.4 |

The experimental error is about ±10%.

*The poly(orthoester) used was a copolymer of cis-trans cyclohexane dimethanol and 3,9-Bis(ethylidenyl)-2,4,8,10-tetraoxaspiro[5,5]undecane (Detosin).

The active agents (therapeutic agent or other beneficial agents) which can be utilized in accordance with the practice of the invention is not critical. Any agent which can be obtained in a stable form is applicable herein. As to the amount of active agent which can be delivered in accordance with the invention, said amount depends on a variety of factors such as the host, physical attributes, particular disease or disorder being treated and of course the severity of the condition being treated. The amount of active agent utilizable in the invention is known to one skilled in the art and is disclosed in various medical references such as PDR and etc. or could be obtained from suitably designed clinical trials.

Generally, the amount of polymer employed in the practice of the invention ranges from 10% to 99.9% polymer (preferably 20% to 60%) by weight of the drug delivery platform. The remaining portion of the composition contains the medicament and conventional pharmaceutically acceptable excipients.

Representative pharmaceutically acceptable excipients that the drug delivery device may contain are buffering agents and preservatives. Suitable water soluble preservatives which may be employed in the drug delivery device are sodium bisulfite, sodium thiosulfate, ascorbate, benzalkonium chloride, chlorobutanol, thimerosal, phenylmercuric borate, parabens, benzyl alcohol and phenylethanol. These agents may be present in amounts from 0 to 5% by weight. Suitable water soluble buffering agents are alkali or alkali earth carbonates, phosphates, bicarbonates, citrates, borates, acetates, acid anhydrides, succinates and the like, such as sodium phosphate, citrate, borate, acetate, bicarbonate and carbonate. These agents may be present in amounts sufficient to maintain some optimum pH of the system in the range 1 to 9. As such the buffering agent can be as much as 25% on a weight to weight basis of the total composition.

The following examples illustrate the preparation of various drug delivery devices of the invention. The Examples should be construed as illustrations rather than limitations thereof.

EXAMPLE 1

A modified tetrahedron was constructed from four Silastic (polydimethylsiloxane) 15% bariumloaded corners with openings for insertion of the poly(ortho ester) (65/35 HD/tCDM)/polyethylene blend rod (Fig. 1). The completed tetrahedron measured 2 × 2 × 2 cm and fit into a 000 gelatin capsule. A biologically active compound may be incorporated into the erodible rod or the silastic corners.

The poly(ortho ester) (a copolymer of 65% hexane diol, 35% trans-cyclohexane dimethanol with DETOSU 65/35 HD/tCDM)/polyethylene blends were tested in Beagle dogs using x-ray techniques. All dogs were dosed in the fasted state (fasted 18 hours before dosing and thereafter for 36 hours). (See Table IV).

## Table IV

| Material | Percent Retained at 24 hrs in Stomach | N* |
|---|---|---|
| Poly(ortho ester) (65/35 HD/tCDM)/poly-ethylene | | |
| 50/50 Blend | 100 | 4 |
| 60/40 Blend | 67 | 3 |

*Number of trials.

EXAMPLE 2

Following the procedure of Example 1, the rods that formed the tetrahedral configuration of the stick figure were fabricated from Klucel HF (hydroxypropylcellulose)/polyethylene blends. In vivo performance in dogs is shown in Table V.

## Table V

| Material | Percent Retained at 24 hrs in Stomach | N |
|---|---|---|
| Klucel HF/polyethylene | | |
| 75/25 Blend | 100 | 4 |
| 90/10 Blend | 50 | 2 |

## EXAMPLE 3

Following the procedure of Example 1, the rods that formed the tetrahedral configuration of the stick figure were fabricated from poly(ortho ester) (c,tCDM)/polyethylene blends. Results are given in the following tables. In vivo performance in dogs is shown in Table VI.

## Table VI

| Poly(ortho ester) (c,tCDM***)/Polyethylene | Percent Retained at 24 hrs in Stomach | N |
|---|---|---|
| 50/50 | 80 | 5 |
| 65/35 | 80 | 5 |
| 75/25 | 100 | 5 |
| 80/20 | 0 | 4 |
| 85/15 | 25 | 4 |
| 90/10 | 0 | 5 |

*** c,tCDM represents a copolymer of cis,trans cyclo-hexanedimethanol with Detosu.

## EXAMPLE 4

Following the procedure of Example 1, the rods that formed the tetrahedral configuration of the stick figure may be fabricated from blends of poly(ortho ester) (65/35 HD/tCDM)/polypropylene.,

## EXAMPLE 5

Following the procedure of Example 1, the rods that formed the tetrahedral configuration of the stick figure may be fabricated from blends of Hydroxypropylmethylcellulose phthalate (HPMCP)/ polyethylene.

## EXAMPLE 6

Following the procedure of Example 1, the rods that formed the tetrahedral configuration of the stick figure may be fabricated from Eudragit E with methylcellulose.

## EXAMPLE 7

This example demonstrates the utility of a tetrahedral configuration that may be made solely from blends of poly(ortho ester) (65/35 HD/tCDM)/ polyethylene. The completed tetrahedron measures 2 × 2 × 2 cm and fits into a number "0" gelatin capsule for dosing (fig. 1a, b and c). A biologically active compound may be incorporated into the erodible material or otherwise contained in an attached drug reservoir via conventional formulation procedures.

## EXAMPLE 8

Following the procedure of Example 7, the material that formed the tetrahedral shape may be fabricated from a poly(ortho ester) (65/35 HD/tCDM)/ EVA blend.

## EXAMPLE 9

Following the procedure of Example 7, the material that formed the tetrahedral shape may be fabricated from a Klucel HF/polypropylene blend.

## EXAMPLE 10

Studies were performed with Beagle dogs in order to ascertain gastric retention time of the drug delivery device described herein. The parameters investigated were size, shape and erodibility. These devices were administered in gelatin capsules and their position in the gastrointestinal tract determined using x-ray techniques.

The results for disc and 4-lobed planar devices of different dimensions and made from Silastic (Dow Company) medical grade silicone rubber labelled with 15% barium sulfate are shown below.

| Shape | Dimensions | % Retained at 24 hrs. | N* |
|---|---|---|---|
| Disc | 2.5 cm diameter | 67 | 9 |
| 4-lobed figure | 3.2 cm diameter | 90 | 10 |
| | 2.7 cm diameter | 83 | 12 |
| | 2.2 cm diameter | 60 | 10 |

\* Number of trials

These data indicate that the retention of these devices in the stomach is a function of their diameter and that retention for a period of 24 hours is possible using devices which can be folded into gelatin capsules suitable for swallowing.

## EXAMPLE 11

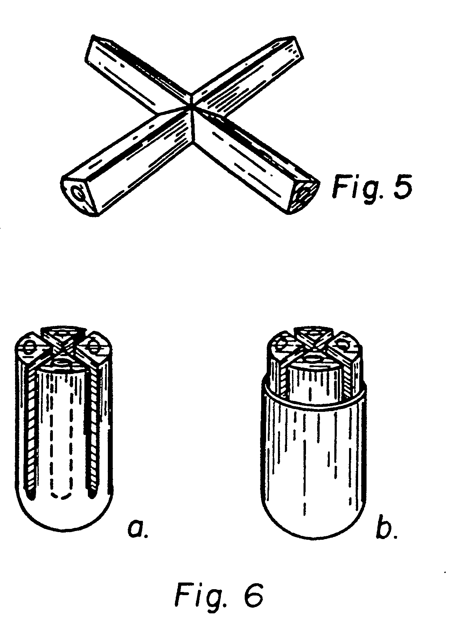

The effect of erosion on the performance of one of the devices is demonstrated in this example. The planar cross form shown in figure 5 was constructed containing segments of rigid, bioacceptable material. For this example segments of spaghetti were inserted into hollows in the limbs of the cross. When the device came into contact with water, the spaghetti segments absorbed water and lost their rigidity. The overall effect was to produce a device which had lost its integrity and which could be more readily expelled from the stomach by the forces of the interdigestive migratory myoelectric complex. The data from one such experiment using Beagle dogs are shown below.

| Shape | % Retained in stomach at 24 hrs. | N |
|---|---|---|
| cross with non-eroding limbs | 100% | 2 |
| cross with eroding limbs (spaghetti) | 71% | 7 |
| cross with empty limbs | 57% | 7 |

The data show that the cross-form with hollow limbs is much less-well retained than the same shape with rigid, non-eroding limbs. The device with initially rigid, but eroding, limbs was intermediate in performance.

EXAMPLE 12

The ring may be fabricated entirely from one material which is erodible or water soluble so that it slowly disintegrates in the confines of the stomach and either breaks up into smaller pieces which are expelled or is no longer able to withstand the contracting forces of the stomach wall and thereby violates criterion (c).

EXAMPLE 13

The ring can be formed from a blend or composite of two or more materials at least one of which is water soluble or erodible which results in predetermined expulsion of the device from the stomach as in Example 12 above.

EXAMPLE 14

Example 12 or 13 where the erodible material is a polyorthoester.

EXAMPLE 15

Example 12 or 13 where the water soluble material is hydroxypropylcellulose.

EXAMPLE 16

The ring can be constructed from a linear portion of erodible or non-erodible material whose ends are joined to form a ring using an erodible or water soluble material. When this joint disintegrates the device will resort to the form of a linear rod or string which can then be expelled from the stomach.

EXAMPLE 17

Example 16 where the erodible point is made of a poly(orthoester).

EXAMPLE 7

Example 16 where the water soluble joint is made of hydroxypropylcellulose.

EXAMPLE 8

Example 12 where the drug is contained in the erodible matrix of the ring.

EXAMPLE 9

Example 12 where the drug is contained in a module which is attached to the ring.

## Claims

1. A gastric retention device comprising a continuous solid-stick figure, a planar figure or ring figure prepared from at least one erodible polymer, said device having the following properties :

a) compressible to a size, allowing its insertion into a retaining capsule or like container designed to dissolve within the stomach after oral ingestion, and suitable for swallowing ;

b) expandable, after dissolution and/or desintegration of said retaining capsule, in its original shape and to size which will prevent passage through a pylorus for a predetermined time ;

c) sufficiently resistant to contractive forces exerced by the stomach, in particular simultaneous forces in two directions acting to reduce the device to a size less large than the pyloric valve, in order to prevent passage through a pylorus for a predetermined time ; and

d) erodible in the presence of gastric juices so that said device after a predetermined time is no longer able to retain or attain the expanded configuration defined in (b) above and/or resist a simultaneous force in two directions as defined in (c) above.

2. The device of Claim 1 wherein said erodible polymer is selected from the group consisting of soluble cellulosic materials, ethylene vinylalcohol, ethylenemaleic anhydride copolymer, polyacrylates, polycaprolactones, inorganic glass based on polyphosphates and fused salts, polyanhydrides, poly(ortho)esters, biodegradable polyrethanes, polyvinyl pyrrolidone, polylactones, polyamides and polypeptides, gelatin and derivatives, polyacrylonitriles, polyesters and combinations thereof.

3. The device of Claim 1, wherein said device is prepared from at least one erodible polymer and at least one non-erodible polymers selected from the group consisting of polyolefins, ethylenevinylacetate copolymers, rubbers, ethylene-vinylalcohol copolymers, polyamides, polyurethanes, polyesters, teflon, non-water-soluble cellulosic and combinations thereof.

4. The device of Claim 1 in stick figure form, wherein said figure is from 1 to 3 cm along each leg of said figure ; wherein said erodible polymer is selected from the group consisting of polyolefins, ethylenevinylacetate copolymer, ethylenevinylalcohol copolymer, poly(ortho)esters, cellulosics, polyanhydrides, polyamides and polypeptides, polyphosphates and fused salt and combinations, composites, blends and copolymers thereof ; wherein the arms of said device have a Tensile Modulus of at least $1 \times 10^3$ to $50 \times 10^6$ psi ; and wherein said predetermined time is from one (1) hour to one (1) year.

5. The device of Claim 4, wherein said polyolefin is polyethylene or polypropylene ; and said cellulosic is hydroxypropylcellulose, cellulose acetate phthalate or ethyl cellulose.

6. The device of Claim 4, wherein said stick figure is from 1.5 to 2.5 cm along each leg of said figure ; said polymer is selected from the group consisting of a mixture of poly(ortho) ester/ polyethylene, a mixture of ethylene/vinylacetate, poly(ortho) ester and polyethylene ; and said predetermined time is from 16 to 48 hours.

7. The device of Claim 6, wherein said polymer is a mixture of poly(ortho ester) and polyethylene.

8. The device of Claim 7, wherein said mixture is 75/25 poly(ortho ester)/polyethylene.

9. The device of Claim 1 wherein said polymer is a poly(ortho) ester.

10. The device of Claim 1 in ring figure form, wherein said ring figure is from 1.6 to 5 cm in diameter, wherein said erodible material is selected from the group consisting of polyolefins, ethylenevinylacetate copolymer, ethylenevinylalcohol copolymer, poly(ortho)esters, cellulosics, polyanhydrides, polyamides and polypeptides, polyphosphates and fused salt and combinations, blends, composites and copolymers thereof ; and wherein said predetermined time is from one (1) hour to one (1) year.

11. The device of Claim 10, wherein said polyolefin is polyethylene or polypropylene ; and said cellulosic is hydroxypropylcellulose, cellulose acetate phthalate or ethyl cellulose.

12. The device of Claim 10, wherein said ring figure is from 3.2 to 3.6 cm in diameter, said material is selected from the group consisting of a mixture of poly(ortho) ester/polyethylene, a mixture of ethylene/vinylacetate, poly(ortho) ester and polyethylene ; and said predetermined time is from 16 to 48 hours.

13. The device of Claim 12, wherein said material is a mixture of poly(ortho ester) and polyethylene.

14. The device of Claim 13, wherein said mixture is a 75/25 weight ratio of poly(ortho ester)/polyethylene.

15. The device of Claim 1 in planar figure form, wherein said planar figure is from 1.6 to 5 cm in diameter; wherein said erodible polymer is selected from the group consisting of polyolefins, ethylenevinylacetate copolymer, ethylenevinylalcohol copolymer, poly(ortho)esters, cellulosics, polyanhydrides, polyamides and polypeptides, polyphosphates and fused salt and combinations, blends and copolymers thereof ; and wherein said predetermined time is from one (1) hour to one (1) year.

16. The device of Claim 15, wherein said polyolefin is polyethylene or polypropylene ; and said cellulosic is hydroxypropylcellulose, cellulose acetate phthalate or ethyl cellulose.

17. The device of Claim 16, wherein said planar figure is from 2 to 4 cm in diameter said polymer is selected from the group consisting of a mixture of poly(ortho) ester/polyethylene, a mixture of ethylene/vinylacetate, poly(ortho) ester and polyethylenep and said predetermined time is from 16 to 48 hours.

18. The device of Claim 17, wherein said polymer is a mixture of poly(ortho ester) and polyethylene.

19. The device of Claim 18, wherein maid mixture is 75/25 poly(ortho ester)/polyethylene.

## Patentansprüche

1. Vorrichtung zur Rückhaltung im Magen, welche ein aus wenigstens einem erodierbaren Polymer hergestelltes kontinuierliches Gebilde in Form eines massiven Stabs, ein planares Gebilde oder ein Ringgebilde umfaßt, wobei die Vorrichtung die folgenden Eigenschaften hat :

a) komprimierbar auf eine Größe, die seine Einführung in eine Rückhaltekapsel oder einen ähnlichen Behälter erlaubt, der eine Gestaltung aufweist, daß er sich nach der oralen Einnahme im Magen auflöst, und zum Verschlukken geeignet ist ;

b) nach der Auflösung und/oder Zersetzung der rückhaltenden Kapsel expandierbar in ihre ursprüngliche Form und Größe, die für eine vorbestimmte Zeit den Durchgang durch den Pförtner verhindert ;

c) hinreichend resistent gegen vom Magen ausgeübte Kontraktionskräfte, insbesondere simultane Kräfte in zwei Richtungen, die auf die Zurückführung der Vorrichtung auf eine Größe hinwirken, die weniger groß ist als der Pförtneraustritt, um für eine vorbestimmte Zeit den Durchgang durch den Pförtner zu verhindern; und

d) erodierbar in Gegenwart von Magensäften, so daß die Vorrichtung nach einer vorbestimmten Zeit nicht länger die in (b) oben definierte expandierte Konfiguration beibehalten oder annehmen kann und/oder einer simultanen Kraft in zwei Richtungen, wie in (c) oben definiert, widerstehen kann.

2. Vorrichtung nach Anspruch 1, worin das erodierbare Polymer aus der aus löslichen Cellulosematerialien, Ethylenvinylalkohol, Ethylen-Maleinsäureanhydrid-Copolymer, Polyacrylaten, Polycaprolactonen, anorganischem Glas auf Basis von Polyphosphaten und geschmolzenen Salzen, Polyanhydriden, Poly(ortho)estern, biodegradierbaren Polyurethanen, Poly- vinylpyrrolidon, Polylactonen, Polyamiden und Polypeptiden, Gelatine und Derivaten, Polyacrylnitrilen, Polyestern und Kombinationen davon bestehenden Gruppe ausgewählt ist.

3. Vorrichtung nach Anspruch 1, worin die Vorrichtung aus wenigstens einem erodierbaren Polymer und wenigstens einem aus der aus Polyolefinen, Ethylen-Vinylacetat-Copolymeren, Gummis, Ethylen-Vinylalkohol-Copolymeren, Polyamiden, Polyurethanen, Polyestern, Teflon, nicht wasserlöslichen Cellulosematerialien und Kombinationen davon bestehenden Gruppe ausgewähltem nicht erodierbarem Polymer hergestellt ist.

4. Vorrichtung nach Anspruch 1 in Stabgebildeform, worin das Gebilde 1 bis 3 cm entlang eines jeden Arms des Gebildes mißt, worin das erodierbare Polymer aus der aus Polyolefinen, Ethylen-Vinylacetat-Copolymer, Ethylen-Vinylalkohol-Copolymer, Poly(ortho)estern, Cellulosematerialien, Polyanhydriden, Polyamiden und Polypeptiden, Polyphosphaten und geschmolzenen Salzen und Kombinationen, Verbundmaterialien, Mischungen und Copolymeren davon bestehenden Gruppe ausgewählt ist, worin die Arme der Vorrichtung einen Zugmodul von wenigstens $1 \times 10^3$ bis $50 \times 10^6$ Psi haben und worin die vorbestimmte Zeit eine (1) Stunde bis ein (1) Jahr beträgt.

5. Vorrichtung nach Anspruch 4, worin das Polyolefin Polyethylen oder Polypropylen ist und das Cellulosematerial Hydroxypropylcellulose, Celluloseacetatphthalat oder Ethylcellulose ist.

6. Vorrichtung nach Anspruch 4, worin das Stabgebilde 1,5 bis 2,5 cm längs jedes Arms des Gebildes mißt, das Polymer aus der aus einer Mischung von Poly(ortho)ester/Polyethylen, einer Mischung aus Ethylen/Vinylacetat, Poly(ortho)ester und Polyethylen bestehenden Gruppe ausgewählt ist und die vorbestimmte Zeit 16 bis 48 Stunden beträgt.

7. Vorrichtung nach Anspruch 6, worin das Polymer eine Mischung aus Poly(ortho)ester und Polyethylen ist.

8. Vorrichtung nach Anspruch 7, worin die Mischung 75/25 Poly(ortho)ester/Polyethylen ist.

9. Vorrichtung nach Anspruch 1, worin das Polymer ein Poly(ortho)ester ist.

10. Vorrichtung nach Anspruch 1 in Form eines Ringgebildes, worin das Ringgebilde einen Durchmesser von 1,6 bis 5 cm aufweist, worin das erodierbare Material aus der aus Polyolefinen, Ethylen-Vinylacetat-Copolymer, Ethylen-Vinylalkohol-Copolymer, Poly(ortho)estern, Cellulosematerialien, Polyanhydriden, Polyamiden und Polypeptiden, Polyphosphaten und geschmolzenen Salzen sowie Kombinationen, Mischungen, Verbundmaterialien und Copolymeren davon bestehenden Gruppe ausgewählt ist und worin die vorbestimmte Zeit eine (1) Stunde bis ein (1) Jahr beträgt.

11. Vorrichtung nach Anspruch 10, worin das Polyolefin Polyethylen oder Polypropylen ist und das Cellulosematerial Hydroxypropylcellulose, Celluloseacetatphthalat oder Ethylcellulose ist.

12. Vorrichtung nach Anspruch 10, worin das Ringgebilde einen Durchmesser von 3,2 bis 3,6 cm hat, das Material aus der aus einer Mischung aus Poly(ortho)ester/Polyethylen, einer Mischung aus Ethylen/Vinylacetat, Poly(ortho)ester und Polyethylen bestehenden Gruppe ausgewählt ist und die vorbestimmte Zeit 16 bis 48 Stunden beträgt.

13. Vorrichtung nach Anspruch 12, worin das Material eine Mischung aus Poly(ortho)ester und Polyethylen ist.

14. Vorrichtung nach Anspruch 13, worin die Mischung ein 75/25Gewichtsverhältnis von Poly(ortho)ester/Polyethylen ist.

15. Vorrichtung nach Anspruch 1 in Form eines planaren Gebildes, worin das planare Gebilde einen Durchmesser von 1,6 bis 5 cm hat, worin das erodierbare Polymer aus der aus Polyolefinen, Ethylen-Vinylacetat-Copolymer, Ethylen-Vinylalkohol-Copolymer, Poly(ortho)estern, Cellulosematerialien, Polyanhydriden, Polyamiden und Polypeptiden, Polyphosphaten und geschmolzenen Salzen sowie Kombinationen, Mischungen und Copolymeren davon bestehenden Gruppe ausgewält ist und worin die vorbestimmte Zeit eine (1) Stunde bis ein (1) Jahr beträgt.

16. Vorrichtung nach Anspruch 15, worin das Polyolefin Polyethylen oder Polypropylen ist und das Cellulosematerial Hydroxypropylcellulose, Celluloseacetatphthalat oder Ethylcellulose ist.

17. Vorrichtung nach Anspruch 16, worin das planare Gebilde einen Durchmesser von 2 bis 4 cm hat und das Polymer aus der aus einer Mischung von Poly(ortho)ester/Polyethylen, einer Mischung aus Ethylen/Vinylacetat, Poly(ortho)ester und Polyethylen bestehenden Gruppe ausgewählt ist und die vorbestimmte Zeit 16 bis 48 Stunden beträgt.

18. Vorrichtung nach Anspruch 17, worin das Polymer eine Mischung aus Poly(ortho)ester und Polyethylen ist.

19. Vorrichtung nach Anspruch 18, worin die Mischung 75/25 Poly(ortho)ester/Polyethylen ist.


## Revendications

1. Dispositif à rétention gastrique ayant une forme continue à base de bâtonnet plein, une forme plane ou une forme annulaire, préparé à partir d'au moins un polymère érodable, ledit dispositif ayant les propriétés suivantes :

a) compressible à une taille permettant son insertion dans une capsule de rétention ou un récipient semblable conçus pour se dissoudre dans l'estomac après l'ingestion orale et appropriés à la déglutition ;

b) expansible, après dissolution et/ou désintégration de ladite capsule de rétention, à sa forme et à sa taille d'origine empêchant le passage à travers un pylore pendant un temps prédéterminé ;

c) suffisamment résistant aux forces de contraction exercées par l'estomac, en particulier aux forces simultanées dans deux directions agissant pour réduire le dispositif à une taille inférieure à la valvule pylorique, pour empêcher le passage à travers le pylore pendant un temps prédéterminé ; et

d) érodable en présence de sucs gastriques pour que ledit dispositif, après un temps prédéterminé, ne puisse plus conserver ou prendre la configuration expansée définie en b) ci-dessus et/ou résister à des forces simultanées dans deux directions comme défini en c) ci-dessus.

2. Dispositif selon la revendication 1, dans lequel ledit polymère érodable est choisi dans le groupe constitué par les matières cellulosiques solubles, un copolymère d'éthylène et d'alcool vinylique, un copolymère d'éthylène et d'anhydride maléique, les polyacrylates, les polycaprolactones, un verre minéral à base de polyphosphates et de sels fondus, les polyanhydrides, les poly(ortho-esters), les polyuréthannes biodégradables, la polyvinylpyrrolidone, les polylactones, les polyamides et les polypeptides, la gélatine et ses dérivés, les polyacrylonitriles, les polyesters et leurs combinaisons.

3. Dispositif selon la revendication 1, où ledit dispositif est préparé à partir d'au moins un polymère érodable et d'au moins un polymère non érodable choisi dans le groupe constitué par les polyoléfines, les copolymères d'éthylène-acétate de vinyle, les caoutchoucs, les copolymères d'éthylène-alcool vinylique, les polyamides, les polyuréthannes, les polyesters, le Téflon, les matières cellulosiques non solubles dans l'eau et leurs combinaisons.

4. Dispositif selon la revencication 1 ayant une forme à base de bâtonnet, dans lequel chaque branche de ladite forme est longue de 1 à 3 cm ; ledit polymère érodable est choisi dans le groupe constitué par les polyofélines, un copolymère d'éthylène et d'acétate de vinyle, un copolymère d'éthylène et d'alcool vinylique, les poly(ortho-esters), les matières cellulosiques, les polyanhydrides, les polyamides et les polypeptides, les polyphosphates et un sel fondu et leurs combinaisons, composites, mélanges et copolymères ; les bras dudit dispositif ont un module de tension d'au moins 0,7 $\times$ 10³ à 34 $\times$ 10⁶ N/cm² (1 $\times$ 10³ à 50 $\times$ 10⁶ psi) ; et ledit temps prédéterminé est de une (1) heure à un (1) an.

5. Dispositif selon la revendication 4, dans lequel ladite polyoléfine est un polyéthylène ou un polypropylène ; et ladite matière cellulosique est l'hydroxypropylcellulose, l'acétophtalate de cellulose ou l'éthylcellulose.

6. Dispositif selon la revendication 4, dans lequel chaque branche de ladite forme à base de bâtonnet est longue de 1,5 à 2,5 cm ; ledit polymer est choisi parmi un mélange de poly(ortho)ester/ polyéthylène, un mélange d'éthylène/acétate de vinyle, un poly(ortho-ester) et un polyéthylène ; et ledit temps prédéterminé est de 16 à 48 heures.

7. Dispositif selon la revendication 6, dans lequel ledit polymère est un mélange de poly(ortho-ester) et de polyéthylène.

8. Dispositif selon la revendication 7, dans lequel ledit mélange est un mélange 75/25 de poly(ortho-ester)/polyéthylène.

9. Dispositif selon la revendication 1, dans lequel ledit polymère est un poly(ortho-ester).

10. Dispositif selon la revendication 1 ayant une forme annulaire, ladite forme annulaire ayant un diamètre de 1,6 à 5 cm, ladite matière érodable est choisie dans le groupe constitué par les polyoléfines, un copolymère d'éthylène et d'acétate de vinyle, un copolymère d'éthylène et d'alcool vinylique, les poly(ortho-esters), les matières cellulosiques, les polyanhydrides, les polyamides et les polypeptides, les polyphosphates et un sel fondu et leurs combinaisons, mélanges, composites et copolymères ; et ledit temps prédéterminé est de une (1) heure à un (1) an.

11. Dispositif selon la revendication 10, dans lequel ladite polyoléfine est un polyéthylène ou un polypropylène ; et ladite matière cellulosique est l'hydroxypropylcellulose, l'acétophtalate de cellulose ou l'éthylcellulose.

12. Dispositif selon la revendication 10, dans lequel ladite forme annulaire a un diamètre de 3,2 à 3,6 cm, ladite matière est choisie parmi un mélange de poly(ortho-ester)/polyéthylène, un mélange d'éthylène/acétate d'éthyle, un poly(ortho-ester) et un polyéthylène ; et ledit temps prédéterminé est de 16 à 48 heures.

13. Dispositif selon la revendication 12, dans lequel ladite matière est un mélange de poly(ortho-ester) et de polyéthylène.

14. Dispositif selon la revendication 13, dans lequel ledit mélange est un mélange 75/25 en poids de poly(ortho-ester) et de polyéthylène.

15. Dispositif selon la revendication 1 sous une forme plane, ladite forme plane ayant un diamètre de 1,6 à 5 cm ; ledit polymère érodable est choisi dans le groupe consitués par les polyéfines, un copolymère d'éthylène et d'acétate de vinyle, un copolymère d'éthylène et d'alcool vinylique, les poly(ortho-esters), les matières cellulosiques, les polyanhydrides, les polyamides et les polypeptides, les polyphosphates et un sel fondu et leurs combinaisons, mélanges et copolymères ; et ledit temps prédéterminé est de une (1) heure à un (1) an.

16. Dispositif selon la revendication 15, dans lequel ladite polyoléfine est un polyéthylène ou un polypropylène ; et ladite matière cellulosique est l'hydroxypropylcellulose, l'acétophtalate de cellulose ou l'éthylcellulose.

17. Dispositif selon la revendication 16, dans lequel ladite forme plane a un diamètre de 2 à 4 cm, ledit polymère est choisi parmi un mélange de poly(ortho-ester)/polyéthylène, un mélange d'éthylène/acétate de vinyle, un poly(ortho)ester et un polyéthylène ; et ledit temps prédéterminé est de 16 à 48 heures.

18. Dispositif selon la revendication 17, dans lequel ledit polymère est un mélange de poly(ortho-ester) et de polyéthylène.

19. Dispositif selon la revendication 18, dans lequel ledit mélange est un mélange 75/25 de poly(ortho-ester)/polyéthylène.

*Fig. I*

Fig. 1(a)

Fig. 1 (b)

Fig. 1 (c)

Fig. 2

a.    b.

Fig. 2

Fig. 3

a.   b.

Fig. 4

Fig. 5

a.

b.

Fig. 6

Fig. 7

a.

b.

Fig. 8